# FASCICULE DE BREVET EUROPEEN

(11) **EP 3 808 292 B1**
(45) Date de publication et mention de la délivrance du brevet: **10.08.2022**
(21) Numéro de dépôt: 20195190.2
(22) Date de dépôt: 09.09.2020
(51) Int. Cl.: A61B 17/50, A61B 18/00

(54) **INSTRUMENT PERMETTANT LA NEUTRALISATION ET LE RETRAIT DE TIQUES FIXEES SUR LA PEAU**
INSTRUMENT ZUR NEUTRALISIERUNG UND ENTFERNUNG VON AUF DER HAUT FESTSITZENDEN ZECKEN
INSTRUMENT ALLOWING THE NEUTRALISING AND REMOVAL OF TICKS ATTACHED TO THE SKIN

(30) Priorité: 17.10.2019 FR 1911646
(43) Date de publication de la demande: 21.04.2021
(73) Titulaire: Domes Pharma, 75014 Paris (FR)
(72) Inventeur: CHAUDER, Anne, 63100 CLERMONT-FERRAND (FR)
(74) Mandataire: Cabinet Laurent & Charras

(56) Documents cités:
- EP-A1- 3 072 464
- WO-A1-2004/054457
- DE-B3-102011 114 360
- US-A- 6 100 501

## Description

### DOMAINE TECHNIQUE

L'invention se rattache au secteur technique des instruments paramédicaux permettant le retrait de parasites fixés sur la peau d'un être humain ou d'un être animal.

Plus précisément, l'invention concerne un instrument permettant la neutralisation de tiques fixées sur la peau au moyen d'une décharge électrique, et permettant également le retrait des tiques.

Les tiques sont des insectes hématophages se fixant sur la peau d'êtres humains ou animaux grâce à un rostre situé à l'extrémité de la tête, ce rostre leur permettant de prélever du sang sur l'être vivant et d'injecter des produits toxiques responsables de maladies, telle que la maladie de Lyme chez l'homme. Il est donc nécessaire de retirer la tique en évitant cette injection et en évitant que le rostre reste ancré dans la peau.

### ART ANTERIEUR

Le document WO 2007/088264 décrit un instrument permettant de neutraliser et retirer des tiques fixées sur la peau d'un animal ou d'un humain. L'instrument comprend un corps tubulaire formé de deux pièces, une des deux pièces du corps se prolongeant par une demi-sphère. Cette demi-sphère vient en complémentarité avec une seconde demi-sphère présente sur une pièce articulée avec le corps de l'instrument, formant ainsi une cage sphérique pour emprisonner le corps de la tique.

La cage sphérique est formée de telle manière à former un orifice de taille fixe pour le maintien de la tique au niveau de la jonction corps/tête de la tique.

Cet instrument est également doté d'un générateur piézoélectrique relié à des fils conducteurs apte à délivrer une tension électrique létale pour la tique. Les arcs électriques générés par le générateur piézoélectrique vont être contenus dans la cage sphérique.

L'inconvénient de cet instrument réside dans le fait qu'il nécessite la présence d'une pièce complémentaire articulée, essentielle pour former la cage sphérique, impliquant des coûts de fabrication et de montage substantiels. Par ailleurs, les arcs électriques permettant de neutraliser la tique contenue dans la sphère, sont instables et non contrôlables. La neutralisation de la tique est donc incertaine puisque rien n'assure qu'un arc électrique va être en contact avec le corps de la tique.

L'autre inconvénient de cet instrument est qu'il ne présente pas de moyens de préhension et de maintien adaptés de la tique. En effet, l'orifice fixe formé par la cage sphérique est par définition non adaptable à la taille de la tique, ce qui peut conduire à enserrer trop fortement ladite tique, avec le risque d'injecter les produits toxiques dans le corps de l'être humain ou de l'animal. A l'inverse, l'orifice peut être de taille trop importante par rapport à celle du rostre de sorte que la tique peut bouger. Il s'ensuit que la tique peut se retrouver en dehors du champ de l'arc électrique avec le risque dans ce cas, que l'utilisateur ressente une décharge électrique sur la peau.

Le document WO 96/38095 divulgue un instrument de forme générale recourbée présentant une fourche à son extrémité, cette fourche permettant d'enserrer la tique au niveau de la jonction corps/tête de la tique afin d'effectuer une rotation permettant de la retirer.

La fourche de cet instrument est formée par deux dents, disposées de telle façon à réaliser une fente pour enserrer la tique. Cette fente présente des bords droits, formant un angle d'ouverture unique.

Ce second instrument ne permet pas d'avoir la certitude que la tique n'a pas injecté de produit toxique dans la mesure où elle n'est pas neutralisée avant retrait.

De plus, la fente de cet instrument formant un angle d'ouverture fixe, l'instrument n'est pas non plus adapté à toute taille de tique. Cela peut conduire à comprimer trop fortement les tiques de grande taille et donc entrainer l'injection de produits toxiques à l'intérieur du corps. C'est la raison pour laquelle le produit est vendu sur le marché en plusieurs modèles pour s'adapter aux tiques en fonction de leur taille.

Le document US6,100,501A montre un instrument pour enlever une tique comportant une fente en V ouverte apte à enserrer une tique et générateur d'électricité destiné à produite une chaleur intense qui obligera la tique à se détacher de la peau.

Le document EP 3 072 464 A1 montre une fabrication en deux demie-coques pour un instrument permettant de neutraliser et retirer des tiques.

### EXPOSE DE L'INVENTION

L'un des buts de l'invention est donc de remédier aux problèmes précités en fournissant un instrument unique permettant la neutralisation d'une tique en s'assurant que celle-ci soit par la suite retirée dans son intégralité, et ce, quelle que soit sa taille.

À cet effet, il a été mis au point un instrument permettant de neutraliser et retirer des tiques fixées sur la peau d'un animal ou d'un humain, ledit instrument comprenant :
- un corps tubulaire formé d'une première pièce et d'une seconde pièce, chaque pièce s'étendant longitudinalement et formant entre elles un volume clos, la première pièce du corps se prolongeant à l'une de ses extrémités par un élément formant cuillère orientée en direction de la seconde pièce,
- un générateur d'électricité relié à des moyens de neutralisation apte à délivrer une tension électrique létale pour la tique,
- des moyens d'activation du générateur d'électricité.

Selon l'invention, l'élément formant cuillère est pourvu d'une fente ouverte s'étendant perpendiculairement par rapport à l'axe longitudinal du corps et apte à enserrer une tique fixée sur la peau d'un animal ou d'un humain.

De cette manière, l'instrument permet de procéder au retrait de la tique en toute sécurité grâce à une première étape de neutralisation conduisant la tique à ne plus être en capacité d'injecter les produits toxiques, et une seconde étape de retrait de la tique effectuée grâce à la fente.

Selon l'invention, le corps tubulaire peut se présenter sous une forme tubulaire de section elliptique, circulaire ou carrée, ou tout autre forme sans sortir du cadre de l'invention.

Selon une forme de réalisation particulière, la fente comprend deux sections distinctes, une première section se situant au niveau de l'ouverture de la fente ; une seconde section située dans le prolongement de la première section, lesdites sections présentant chacune un angle d'ouverture compris entre 2° et 18°, préférentiellement entre 4° et 14°.

Cette forme de réalisation permet à l'instrument de s'adapter à des tiques de toute taille. En effet, une fente avec un angle d'ouverture unique est problématique, et peut même s'avérer nocif puisqu'en cas d'incompatibilité avec la taille de la tique, cette fente à angle unique pourrait comprimer excessivement le corps de la tique et la conduire à injecter les produits toxiques qu'elle contient à l'intérieur de l'organisme. Au contraire, si la taille de la fente est supérieure à celle de la tique, la tique ne pourra pas être retirée.

De manière plus détaillée, la fente comprend une première section, préférentiellement d'un angle d'ouverture compris entre 11° et 18°, avantageusement d'un angle d'ouverture de 14°; et une seconde section étroite, préférentiellement d'un angle d'ouverture compris entre 2° et 10°, avantageusement de 4°.

Selon un mode de réalisation particulier le fond de la fente est arrondi.

Cette caractéristique présente l'avantage d'avoir une meilleure préhension de la tique permettant d'effectuer son retrait, et par ailleurs permet de mieux enserrer la tique au niveau de la jonction corps/rostre de la tique. En effet, le fond arrondi de la fente entraine un retrait optimal dans la mesure où le mouvement implique une rotation de la tique au niveau de cette jonction. Selon un mode de réalisation préférentiel, la première section s'étend sur une longueur comprise entre 50% et 80% de la longueur totale de la fente, et préférentiellement sur une longueur de 60% ; tandis que la seconde section s'étend sur une longueur comprise entre 20% et 50% de la longueur totale de la fente, préférentiellement sur une longueur de 40%.

Selon une variante optionnelle, les bords de la fentes sont biseautés, avantageusement sur toute sa longueur de manière à épouser au mieux la forme de la tique.

Préférentiellement, les moyens de neutralisation se présentent sous la forme d'au moins deux fils électriques, chaque fil présentant une première terminaison connectée au générateur d'électricité et une seconde terminaison libre, les secondes terminaisons étant positionnées dans l'élément formant cuillère orientée en direction de la seconde pièce, de part et d'autre de la fente.

La présence et la disposition particulière de ces fils électriques au niveau de la fente permet au courant généré par le générateur électrique de former un seul arc électrique au niveau du corps de la tique. Ainsi, on évite la formation de plusieurs arcs électriques non nécessaires, en s'assurant que le seul arc électrique généré est bien en contact avec le corps de la tique. Il s'ensuit que l'utilisateur n'a aucun risque de recevoir une décharge électrique.

De plus, les secondes terminaisons sont préférentiellement coaxiales selon un axe perpendiculaire à l'axe longitudinal du corps, de sorte à assurer la stabilité et la direction de l'arc électrique.

Les moyens de neutralisation peuvent également se présenter sous la forme de pièces métalliques capables de former un arc électrique lorsqu'elles sont reliées au générateur d'électricité, ou tout autre moyen de neutralisation sans toutefois sortir du cadre de l'invention.

Selon une forme de réalisation particulière, la seconde pièce du corps se prolonge à une de ses extrémités par un élément complémentaire dont la forme se superpose sur toute sa surface avec la surface correspondante de l'élément formant cuillère, à l'exception de la fente. De manière avantageuse, cet élément complémentaire est également en forme de cuillère.

L'élément complémentaire peut également, selon une variante optionnelle, avoir une forme qui ne se superpose que partiellement avec la surface correspondante de l'élément formant cuillère.

Préférentiellement, chaque pièce du corps est pourvu d'agencements aptes à maintenir les moyens de neutralisation en position une fois les deux pièces du corps rendues solidaires.

Ces agencements sont avantageux afin de maintenir les secondes terminaisons des fils de façon perpendiculaire par rapport à l'axe longitudinal du corps, dans le but d'avoir un arc électrique stable au niveau du corps de la tique.

Selon une forme de réalisation particulière, les moyens d'activation comprennent au moins un bouton poussoir solidaire du générateur d'électricité.

Ce bouton poussoir permet ainsi d'activer le générateur d'électricité une fois la tique enserrée et immobilisée dans la fente.

Les moyens d'activation peuvent être de tout type appropriés sans sortir du cadre de l'invention, tel qu'un interrupteur ou autre mécanisme adéquat.

Selon un mode de réalisation préférentiel, le corps est solidaire d'un organe de pression situé au niveau d'une seconde extrémité du corps, apte à interagir avec le bouton poussoir du générateur d'électricité, afin d'améliorer l'ergonomie de l'instrument.

Selon une forme de réalisation particulière, le générateur d'électricité est un générateur piézoélectrique dans le but d'éviter un rechargement du générateur au moyen de piles ou de batteries.

### DESCRIPTION DES FIGURES

La figure 1 est une coupe longitudinale de l'instrument selon l'invention vu de face.
La figure 2 est une représentation schématique en coupe similaire à celle de la figure 1, ou l'instrument est représenté vu de côté selon le plan AA' de la figure 1.
La figure 3 est une coupe longitudinale la première pièce du corps de l'instrument selon l'invention selon le plan AA' de la figure 1.
La figure 4 est une représentation schématique similaire à celle de la figure 3, illustrant la seconde pièce du corps selon le plan AA' de la figure 1.
La figure 5 est une représentation en perspective illustrant la cuillère de préhension de l'instrument selon l'invention.
La figure 6 illustre la cuillère du dispositif de l'invention vue de dessous, selon la flèche VI
La figure 7 est une représentation schématique illustrant la fente de la cuillère de l'instrument selon l'invention, vue de dessous, selon la flèche VI.

### DESCRIPTION DETAILLEE DE L'INVENTION

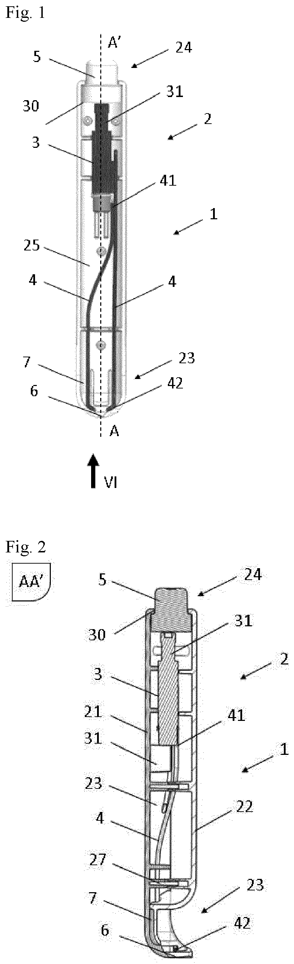

En référence aux figures 1 à 4, l'invention concerne un instrument (1) permettant de neutraliser et de retirer des tiques fixées sur la peau d'un animal ou d'un humain. L'instrument (1) comprend un corps (2) tubulaire, de section elliptique et en matière polymère, formé d'une première pièce (21) et d'une seconde pièce (22) destinées à coopérer entre elles, chaque pièce s'étendant longitudinalement et formant un volume clos (25) lorsqu'elles sont assemblées.

L'une des extrémités de la première pièce (21) du corps (2) se prolonge par un élément (26) formant une cuillère, solidaire d'un élément complémentaire (27) prolongeant l'une des extrémités de la seconde pièce (22) formant également une cuillère.

La forme en cuillère de l'élément (26) en prolongement de la première pièce (21) se comprend ici comme une forme globalement concave, c'est-à-dire dont l'axe de courbure est perpendiculaire à l'axe longitudinal et perpendiculaire au plan AA' tel que représenté sur la figure 2.

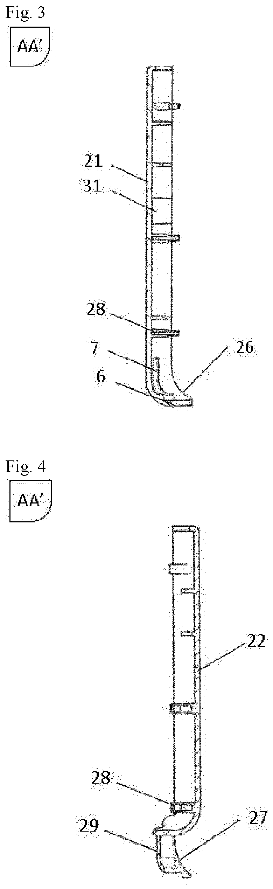

En référence aux figures 3 et 4, l'instrument (1) comprend des moyens de fixation (28) permettant aux deux pièces (21, 22) qui constituent le corps (2) de l'instrument (1) de s'assembler, afin de constituer le volume clos (25) du corps (2).

En référence à l'ensemble des figures, la forme du corps (2) assemblé se présente sous une forme tubulaire de section elliptique.

Toujours en référence aux figures 3 et 4, la première pièce (21) et la seconde pièce (22) du corps (2) présentent respectivement sur une de leur extrémités un élément (26) et un élément complémentaire (27) de forme sensiblement similaire en cuillère, ce qui permet de donner la forme globale en cuillère de la première extrémité (23) de l'instrument (1).

Les agencements (7) permettent de maintenir les fils électriques (4).

Plus précisément, la seconde pièce (22) du corps (2) se prolonge par un élément complémentaire (27) présentant un profil interne concave (29). Cette forme concave vient en complémentarité avec la forme également concave de l'élément (26) situé sur la première pièce (21) du corps (2).

Selon la figure 2 et 3, la surface interne du corps (2) est pourvue d'éléments de soutien (31) du générateur d'électricité (3) afin d'éviter son déplacement et de faire appui lors de l'activation du générateur d'électricité (3).

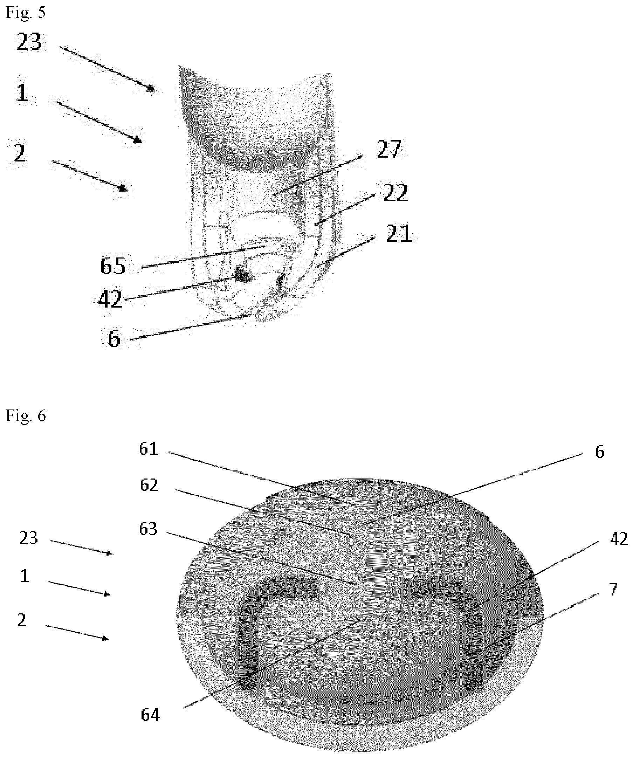

En référence à la figure 5, l'élément (26) de la première pièce (21) du corps (2) en forme de cuillère présente une surface externe de forme sensiblement sphérique.

En référence aux figures 1 et 2, l'instrument (1) comprend également un générateur d'électricité (3) relié à des fils électriques apte à délivrer une tension électrique létale pour la tique, ainsi que des moyens d'activation du générateur d'électricité (3).

Le générateur d'électricité (3) est activable en exerçant une pression sur le bouton poussoir (31), ou en exerçant une pression sur un organe de pression (5) qui agira lui-même sur le bouton poussoir (31). Cet organe de pression (5) est disposé dans un orifice (30) aménagé dans la seconde extrémité (24) du corps (2).

Toujours selon les figures 1 et 2, les moyens de neutralisation se présentent sous la forme de fils électriques (4).

Ces fils électriques (4), aptes à acheminer le courant électrique jusqu'à la première extrémité (23) du corps (2) lors de l'activation du générateur d'électricité (3), présentent une première terminaison (41) reliée directement au générateur d'électricité (3), tandis que la seconde terminaison (42) est insérée dans les agencements (7) de sorte à ce qu'elles soient maintenues.

Plus précisément, la figure 1 montre les agencements (7) dans la première extrémité (23) du corps (2), prévus de part et d'autre de la fente (6) et aptes à accueillir les secondes terminaisons (42) des deux fils électriques (4) pour les maintenir et les acheminer de façon précise jusqu'à proximité de la fente (6).

En référence aux figures 2 et 5, les secondes terminaisons (42) des fils électriques (4) sont positionnées de part et d'autre de la fente (6) de manière sensiblement coaxiale selon un axe perpendiculaire à l'axe longitudinal de la fente (6).

Le positionnement de ces secondes terminaisons (42) selon un axe coaxial est idéal afin de former un arc électrique au niveau du corps de la tique, l'arc électrique étant formé au-dessus de la fente (6) en direction de la seconde extrémité (24) du corps (2).

La figure 5 permet de mieux visualiser la première extrémité (23) en forme de cuillère de l'instrument (1), ainsi que les deux terminaisons (42) des deux fils électriques (4) se faisant face, lesquels sont maintenus par les agencements (7).

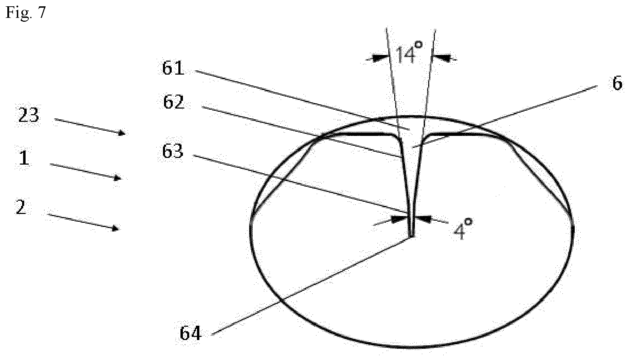

En référence aux figures 6 et 7, l'élément (26) formant cuillère est pourvu d'une fente (6) ouverte s'étendant perpendiculairement par rapport à l'axe longitudinal du corps (2) et apte à enserrer puis retirer une tique fixée sur la peau d'un animal ou d'un humain.

Plus précisément et en référence à la figure 7, la fente (6) comprend deux sections distinctes, la première section (62) se situant au niveau de l'ouverture (61) de la fente (6) ; suivie d'une seconde section plus étroite (63). Lesdites sections (62, 63) présentent un angle d'ouverture compris entre 4° et 14°. Le fond (64) de la fente (6) est de forme arrondie.

De manière plus détaillée, la première section (62) présente un angle d'ouverture de 14° et s'étend sur environ 60% de la longueur totale de la fente (6), tandis que la seconde section présente un angle d'ouverture de 4° et s'étend sur environ 40% de la longueur totale de la fente.

Ces deux sections (62, 63) distinctes, pourvues dans ce mode de réalisation de bords biseautés, permettent un meilleur enserrement de la tique au niveau de la jonction entre le corps et le rostre de la tique ; elle permet également de s'adapter à toute taille de tique.

En référence à la figure 5, l'opération d'enserrement de la tique permet de maintenir le corps de la tique au sein de la première extrémité (23) formant cuillère de l'instrument (1), plus précisément dans le creux de la cuillère, au niveau des deux terminaisons (42) des fils électriques (4). Cet enserrement optimisé de la tique permet de positionner son corps au niveau de l'arc électrique rendant ainsi sa neutralisation certaine.

On observe également sur la figure 5 une échancrure (65) présente sur l'élément complémentaire (27) de la seconde pièce (22) du corps (2). Cette échancrure (65) est nécessairement plus large que la fente (6) pour ne pas compromettre la fonction de cette dernière, à savoir la préhension et le maintien de la tique.

Cette échancrure (65) présente sur la seconde pièce (22) du corps (2) est dans la continuité supérieure de la fente (6), pour un maintien optimal de la tique.

En référence aux figures 6 et 7, le fond (64) de la fente (6) est arrondi, de manière à assurer à la fois un meilleur maintien et une préhension adéquate au niveau de la jonction entre le rostre et le corps de la tique lors de son retrait, après sa neutralisation.

## Revendications

1. Instrument (1) permettant de neutraliser et retirer des tiques fixées sur la peau d'un animal ou d'un humain, ledit instrument (1) comprenant :
- un corps (2) tubulaire formé d'une première pièce (21) et d'une seconde pièce (22), chaque pièce s'étendant longitudinalement et formant entre elles un volume clos (25), la première pièce (21) du corps (2) se prolongeant à l'une de ses extrémités par un élément (26) formant cuillère orientée en direction de la seconde pièce,
- un générateur d'électricité (3) relié à des moyens de neutralisation apte à délivrer une tension électrique létale pour la tique,
- des moyens d'activation du générateur d'électricité (3), l'élément (26) formant cuillère étant pourvu d'une fente (6) ouverte s'étendant perpendiculairement par rapport à l'axe longitudinal du corps (2) et apte à enserrer une tique fixée sur la peau d'un animal ou d'un humain.

2. Instrument (1) selon la revendication 1, **caractérisé en ce que** la fente (6) comprend deux sections distinctes (62,63), une première section (62) se situant au niveau de l'ouverture (61) de la fente (6) ; une seconde section (63) située dans le prolongement de la première section (62), lesdites sections (62, 63) présentant un angle d'ouverture compris entre 2° et 18°, préférentiellement entre 4° et 14°.

3. Instrument (1) selon la revendication 2, **caractérisé en ce que** la première section (62) s'étend sur une longueur comprise entre 50% et 80% de la longueur totale de la fente (6), tandis que la seconde section (63) s'étend sur une longueur comprise entre 20% et 50% de la longueur totale de la fente (6).

4. Instrument (1) selon l'une des revendications précédentes, **caractérisé en ce que** le fond (64) de la fente (6) est arrondi.

5. Instrument (1) selon l'une des revendications précédentes, **caractérisé en ce que** les bords de la fente sont biseautés.

6. Instrument (1) selon une ou plusieurs des revendications précédentes, **caractérisé en ce que** les moyens de neutralisation se présentent sous la forme d'au moins deux fils électriques (4), chaque fil présentant une première terminaison (41) connectée au générateur d'électricité (3) et une seconde terminaison (42) libre, les secondes terminaisons (42) étant positionnées à l'intérieur de la première extrémité (23) du corps (2) formant cuillère, de part et d'autre de la fente (6).

7. Instrument (1) selon une ou plusieurs des revendications précédentes, **caractérisé en ce que** la seconde pièce (22) du corps (2) se prolonge à une de ses extrémités (23) par un élément complémentaire (27) dont la forme se superpose sur toute sa surface avec la surface correspondante de l'élément (26) formant cuillère, à l'exception de la fente (6).

8. Instrument (1) selon une ou plusieurs des revendications précédentes, **caractérisé en ce que** chaque pièce du corps (2) est pourvu d'agencements (7) aptes à maintenir les moyens de neutralisation en position une fois les deux pièces (21, 22) du corps (2) rendues solidaires.

9. Instrument (1) selon une ou plusieurs des revendications précédentes 1, **caractérisé en ce que** les moyens d'activation comprennent au moins un bouton poussoir (31) solidaire du générateur d'électricité (3)

10. Instrument (1) selon une ou plusieurs des revendications précédentes, **caractérisé en ce que** le corps (2) est solidaire d'un organe de pression (5) situé au niveau d'une seconde extrémité (24) du corps (2), apte à interagir avec le bouton poussoir (31) du générateur d'électricité (3).

11. Instrument (1) selon une ou plusieurs des revendications précédentes, **caractérisé en ce que** le générateur d'électricité (3) est un générateur piézoélectrique.

## Patentansprüche

1. Instrument (1) zur Neutralisierung und Entfernung von Zecken, die auf der Haut eines Tieres oder Menschen haften, dieses Instrument (1) umfasst:
- einen rohrförmigen Körper (2), gebildet aus einem ersten Teil (21) und einem zweiten Teil (22), jedes Teil verläuft dabei in Längsrichtung und zwischen sich bilden sie einen geschlossenen Raum (25), das erste Teil (21) des Körpers (2) geht an einem Ende in ein Element (26) über, das einen Löffel bildet, ausgerichtet in Richtung des zweiten Teils.
- einen Stromgenerator (3), verbunden mit Neutralisierungsmitteln, der in der Lage ist eine für die Zecke letale Stromspannung abzugeben,
- Aktivierungsmittel für den Stromgenerator (3),
das den Löffel bildende Element (26) ist mit einem offenen Spalt (6) ausgerüstet, der senkrecht zur Längsachse des Körpers (2) verläuft und eine auf der Haut eines Tiers oder Menschen haftende Zecke einschließen kann.

2. Instrument (1) nach Anspruch 1, **dadurch gekennzeichnet, dass** der Spalt (6) zwei unterschiedliche Abschnitte (62,63) enthält, ein erster Abschnitt befindet sich in Höhe der Öffnung (61) des Spaltes (6); ein zweiter Abschnitt (63) befindet sich in der Verlängerung des ersten Abschnitts (62), dabei weisen diese Abschnitte (62, 63) einen Öffnungswinkel zwischen 2° und 18° auf, vorzugsweise zwischen 4° und 14°.

3. Instrument (1) nach Anspruch 2, **dadurch gekennzeichnet, dass** der erste Abschnitt (62) über eine Länge reicht, die zwischen 50% und 80% der Gesamtlänge des Spaltes (6) beträgt, während der zweite Abschnitt über eine Länge reicht, die zwischen 20% und 50% der Gesamtlänge des Spaltes (6) beträgt.

4. Instrument (1) nach einem der vorangehenden Ansprüche, **dadurch gekennzeichnet, dass** der Boden (64) des Spaltes (6) abgerundet ist.

5. Instrument (1) nach einem der vorangehenden Ansprüche, **dadurch gekennzeichnet, dass** die Ränder des Spaltes abgeschrägt sind.

6. Instrument (1) nach einem oder mehreren der vorangehenden Ansprüche, **dadurch gekennzeichnet, dass** die Neutralisierungsmittel die Form von mindestens zwei elektrischen Drähten (4) haben, jeder Draht weist einen ersten Abschluss (41) auf, der mit dem Stromgenerator (3) verbunden ist und einen zweiten, freien Abschluss (42), die zweiten Abschlüsse (42) sind beiderseits des Spaltes (6) innerhalb des ersten Endstücks (23) des Körpers (2) angeordnet, das einen Löffel bildet.

7. Instrument (1) nach einem oder mehreren der vorangehenden Ansprüche, **dadurch gekennzeichnet, dass** der zweite Teil (22) des Körpers (2) an einem seiner Enden (23) in ein komplementäres Element (27) übergeht, dessen Form auf der gesamten Oberfläche sich über die entsprechende Oberfläche des Elementes (26), das einen Löffel bildet, legt, mit Ausnahme des Spalts (6).

8. Instrument (1) nach einem oder mehreren der vorangehenden Ansprüche, **dadurch gekennzeichnet, dass** jeder Teil des Körpers (2) mit Anordnungen (7) ausgerüstet ist, die in der Lage sind, die Neutralisierungsmittel zu halten, sobald die beiden Teile (21, 22) des Körpers (2) miteinander fest verbunden sind.

9. Instrument (1) nach einem oder mehreren der vorangehenden Ansprüche, **dadurch gekennzeichnet, dass** die Aktivierungsmittel mindestens einen Druckknopf (31) enthalten, der fest mit dem Stromgenerator (3) verbunden ist.

10. Instrument (1) nach einem oder mehreren der vorangehenden Ansprüche, **dadurch gekennzeichnet, dass** der Körper (2) fest mit einem Druckorgan (5) verbunden ist, der in Höhe eines zweiten Endstücks (24) des Körpers (2) angeordnet ist, und in der Lage ist, mit dem Druckknopf (31) des Stromgenerators (3) zu interagieren.

11. Instrument (1) nach einem oder mehreren der vorangehenden Ansprüche, **dadurch gekennzeichnet, dass** der Stromgenerator (3) ein piezoelektrischer Generator ist.

## Claims

1. Instrument (1) allowing the neutralising and removal of ticks attached to the skin of an animal or a human, said instrument (1) comprising:
- a tubular body (2) formed of a first part (21) and of a second part (22), each part extending longitudinally and forming a closed volume (25) therebetween, the first part (21) of the body (2) extending to one of its ends by a spoon-forming element (26) oriented in the direction of the second part,
- an electricity generator (3) connected to neutralising means, capable of delivering an electrical voltage which is lethal for the tick,
- means for activating the electricity generator (3), the spoon-forming element (26) being provided with an open slot (6) extending perpendicularly with respect to the longitudinal axis of the body (2), and capable of sandwiching a tick attached to the skin of an animal or a human.

2. Instrument (1) according to claim 1, **characterised in that** the slot (6) comprises two separate sections (62, 63), a first section being located at the opening (61) of the slot (6); a second section (63) located in the extension of the first section (62), said sections (62, 63) having an opening angle of between 2° and 18°, preferably of between 4° and 14°.

3. Instrument (1) according to claim 2, **characterised in that** the first section (62) extends over a length of between 50% and 80% of the total length of the slot (6), while the second section extends over a length of between 20% and 50% of the total length of the slot (6).

4. Instrument (1) according to one of the preceding claims, **characterised in that** the bottom (64) of the slot (6) is curved.

5. Instrument (1) according to one of the preceding claims, **characterised in that** the edges of the slot are bevelled.

6. Instrument (1) according to one or more of the preceding claims, **characterised in that** the neutralising means are in the form of at least two electrical wires (4), each wire having a first termination (41) connected to the electricity generator (3) and a second free termination (42), the second terminations (42) being positioned inside the first end (23) of the spoon-forming body (2), on either side of the slot (6).

7. Instrument (1) according to one or more of the preceding claims, **characterised in that** the second part (22) of the body (2) extends to one of its ends (23) by a complementary element (27), the shape of which is superposed over the whole of its surface with the corresponding surface of the spoon-forming element (26), except for the slot (6).

8. Instrument (1) according to one or more of the preceding claims, **characterised in that** each part of the body (2) is provided with arrangements (7), capable of holding the neutralising means in position once the two parts (21, 22) of the body (2) are made integral.

9. Instrument (1) according to one or more of the preceding claims, **characterised in that** the activation means comprise at least one pushbutton (31) integral with the electricity generator (3).

10. Instrument (1) according to one or more of the preceding claims, **characterised in that** the body (2) is integral with a pressure member (5) located at a second end (24) of the body (2), capable of interacting with the pushbutton (31) of the electricity generator (3).

11. Instrument (1) according to one or more of the preceding claims, **characterised in that** the electricity generator (3) is a piezoelectric generator.
